# EUROPEAN PATENT APPLICATION

(11) **EP 3 699 168 A1**
(43) Date of publication of application: **26.08.2020**
(21) Application number: 20169132.6
(22) Date of filing: 04.02.2015
(51) Int. Cl.: C07C 69/155, C07C 69/145

(54) **BRANCHED-CHAIN ESTERS AND METHODS OF MAKING AND USING THE SAME**

(30) Priority: 18.02.2014 US 201461941048 P; 19.02.2014 US 201461941726 P
(62) Divisional of application: 15751927.3
(71) Applicant: Elevance Renewable Sciences, Inc., Woodridge, IL 60517 (US)
(72) Inventor: BERTIN, Paul A, Woodridge, IL 60517 (US); SHANER, Courtnay, Woodridge, IL 60517 (US); WEITKAMP, Robin, Woodridge, IL 60517 (US)
(74) Representative: Murgitroyd & Company

(57) **Abstract**

Branched-chain esters and methods of making branched-chain esters are generally disclosed. Various uses of such compounds are also disclosed, including uses in personal care compositions and lubricant compositions. In some embodiments, the branched-chain esters are at least partially derived from a renewable source, such as a natural oil.

## Description

### CROSS-REFERENCE TO RELATED APPLICATIONS

The present divisional application derived from European patent Application No. 15751927.3 (EP3107890) filed 4 February 2015, claims the benefit of priority to United States Provisional Application Nos.: 61/941,048, filed February 18, 2014; and 61/941,726, filed February 19, 2014; both of which are hereby incorporated by reference in their entirety as though fully set forth herein.

### TECHNICAL FIELD

Branched-chain esters and methods of making branched-chain esters are generally disclosed. Various uses of such compounds are also disclosed, including uses in personal care compositions and lubricant compositions. In some embodiments, the branched-chain esters are at least partially derived from a renewable source, such as a natural oil.

### BACKGROUND

Branched-chain compounds, such as Guerbet alcohols, have utility in a variety of contexts. For example, such compounds can serve as components of lubricant compositions. They can also find use in certain personal care items.

The commercial availability of such compounds is limited to those products that can be made readily from available sources. For example, Guerbet alcohols are made by the Guerbet process, and are therefore limited to compounds that can be formed by that process. The range of inputs to the process may also be limited, which can place additional limits on the branched-chain compounds that can be made.

There is also an increasing demand for materials, such as lubricants and personal care items, to employ compounds that are at least partially derived from renewable sources, such as from various natural oils.

Thus, there is a continuing need to discover a broader range of branched-chain compounds that can provide a broader range of properties than the currently available range of branched-chain compounds, such as Guerbet alcohols. And there is a need to develop materials derived from renewable sources.

### SUMMARY

In a first aspect, the disclosure provides branched-chain monoesters (e.g., mono-estolides). Such compounds can, among other uses, be included in a lubricant composition or in a personal care composition. In some embodiments, the branched-chain monoesters are compounds of Formula (I): 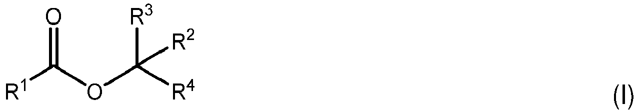 wherein: R¹ is C₃₋₂₄ alkyl or C₃₋₂₄ alkenyl, each of which is optionally substituted; R² is a hydrogen atom or C₁₋₆ alkyl, which is optionally substituted; and R³ and R⁴ are independently C₃₋₂₄ alkyl or C₃₋₂₄ alkenyl, each of which are optionally substituted.

In a second aspect, the disclosure provides a composition that includes one or more of the branched-chain esters of the first aspect. In some embodiments, the composition is a lubricant composition. In some other embodiments, the composition is a personal care composition.

In a third aspect, the disclosure provides methods for making compositions of the second aspect. In some embodiments, the compounds of the first aspect are mixed with a diluent, such as water or a lubricant base oil.

Further aspects and embodiments are provided in the foregoing drawings, detailed description and claims.

### BRIEF DESCRIPTION OF THE DRAWINGS

The following drawings are provided for purposes of illustrating various embodiments of the compositions and methods disclosed herein. The drawings are provided for illustrative purposes only, and are not intended to describe any preferred compositions or preferred methods, or to serve as a source of any limitations on the scope of the claimed inventions.

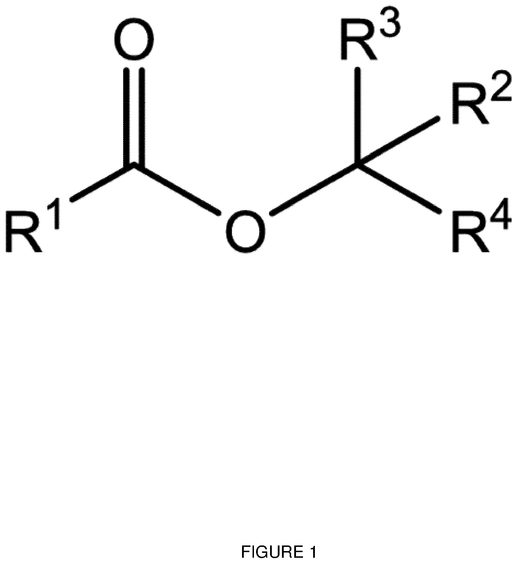

Figure 1 shows a non-limiting example of a compound of certain embodiments disclosed herein, where R¹ is C₃₋₂₄ alkyl or C₃₋₂₄ alkenyl, each of which is optionally substituted; R² is a hydrogen atom or C₁₋₆ alkyl, which is optionally substituted; and R³ and R⁴ are independently C₃₋₂₄ alkyl or C₃₋₂₄ alkenyl, each of which are optionally substituted.

### DETAILED DESCRIPTION

The following description recites various aspects and embodiments of the inventions disclosed herein. No particular embodiment is intended to define the scope of the invention. Rather, the embodiments provide non-limiting examples of various compositions, and methods that are included within the scope of the claimed inventions. The description is to be read from the perspective of one of ordinary skill in the art. Therefore, information that is well known to the ordinarily skilled artisan is not necessarily included.

### Definitions

The following terms and phrases have the meanings indicated below, unless otherwise provided herein. This disclosure may employ other terms and phrases not expressly defined herein. Such other terms and phrases shall have the meanings that they would possess within the context of this disclosure to those of ordinary skill in the art. In some instances, a term or phrase may be defined in the singular or plural. In such instances, it is understood that any term in the singular may include its plural counterpart and vice versa, unless expressly indicated to the contrary.

As used herein, the singular forms "a," "an," and "the" include plural referents unless the context clearly dictates otherwise. For example, reference to "a substituent" encompasses a single substituent as well as two or more substituents, and the like.

As used herein, "for example," "for instance," "such as," or "including" are meant to introduce examples that further clarify more general subject matter. Unless otherwise expressly indicated, such examples are provided only as an aid for understanding embodiments illustrated in the present disclosure, and are not meant to be limiting in any fashion. Nor do these phrases indicate any kind of preference for the disclosed embodiment.

As used herein, "natural oil," "natural feedstock," or "natural oil feedstock" refer to oils derived from plants or animal sources. These terms include natural oil derivatives, unless otherwise indicated. The terms also include modified plant or animal sources (e.g., genetically modified plant or animal sources), unless indicated otherwise. Examples of natural oils include, but are not limited to, vegetable oils, algae oils, fish oils, animal fats, tall oils, derivatives of these oils, combinations of any of these oils, and the like. Representative non-limiting examples of vegetable oils include rapeseed oil (canola oil), coconut oil, corn oil, cottonseed oil, olive oil, palm oil, peanut oil, safflower oil, sesame oil, soybean oil, sunflower oil, linseed oil, palm kernel oil, tung oil, jatropha oil, mustard seed oil, pennycress oil, camelina oil, hempseed oil, and castor oil. Representative non-limiting examples of animal fats include lard, tallow, poultry fat, yellow grease, and fish oil. Tall oils are by-products of wood pulp manufacture. In some embodiments, the natural oil or natural oil feedstock comprises one or more unsaturated glycerides (e.g., unsaturated triglycerides). In some such embodiments, the natural oil feedstock comprises at least 50% by weight, or at least 60% by weight, or at least 70% by weight, or at least 80% by weight, or at least 90% by weight, or at least 95% by weight, or at least 97% by weight, or at least 99% by weight of one or more unsaturated triglycerides, based on the total weight of the natural oil feedstock.

As used herein, "natural oil derivatives" refers to the compounds or mixtures of compounds derived from a natural oil using any one or combination of methods known in the art. Such methods include but are not limited to saponification, fat splitting, transesterification, esterification, hydrogenation (partial, selective, or full), isomerization, oxidation, and reduction. Representative non-limiting examples of natural oil derivatives include gums, phospholipids, soapstock, acidulated soapstock, distillate or distillate sludge, fatty acids and fatty acid alkyl ester (e.g. non-limiting examples such as 2-ethylhexyl ester), hydroxy substituted variations thereof of the natural oil. For example, the natural oil derivative may be a fatty acid methyl ester ("FAME") derived from the glyceride of the natural oil. In some embodiments, a feedstock includes canola or soybean oil, as a non-limiting example, refined, bleached, and deodorized soybean oil (i.e., RBD soybean oil). Soybean oil typically comprises about 95% weight or greater (e.g., 99% weight or greater) triglycerides of fatty acids. Major fatty acids in the polyol esters of soybean oil include saturated fatty acids, as a non-limiting example, palmitic acid (hexadecanoic acid) and stearic acid (octadecanoic acid), and unsaturated fatty acids, as a non-limiting example, oleic acid (9-octadecenoic acid), linoleic acid (9, 12-octadecadienoic acid), and linolenic acid (9,12,15-octadecatrienoic acid).

As used herein, "metathesis catalyst" includes any catalyst or catalyst system that catalyzes an olefin metathesis reaction.

As used herein, "metathesize" or "metathesizing" refer to the reacting of a feedstock in the presence of a metathesis catalyst to form a "metathesized product" comprising new olefinic compounds, i.e., "metathesized" compounds. Metathesizing is not limited to any particular type of olefin metathesis, and may refer to cross-metathesis (i.e., co-metathesis), self-metathesis, ring-opening metathesis, ring-opening metathesis polymerizations ("ROMP"), ring-closing metathesis ("RCM"), and acyclic diene metathesis ("ADMET"). In some embodiments, metathesizing refers to reacting two triglycerides present in a natural feedstock (self-metathesis) in the presence of a metathesis catalyst, wherein each triglyceride has an unsaturated carbon-carbon double bond, thereby forming a new mixture of olefins and esters which may include a triglyceride dimer. Such triglyceride dimers may have more than one olefinic bond, thus higher oligomers also may form. Additionally, in some other embodiments, metathesizing may refer to reacting an olefin, such as ethylene, and a triglyceride in a natural feedstock having at least one unsaturated carbon-carbon double bond, thereby forming new olefinic molecules as well as new ester molecules (cross-metathesis).

As used herein, "hydrocarbon" refers to an organic group composed of carbon and hydrogen, which can be saturated or unsaturated, and can include aromatic groups. The term "hydrocarbyl" refers to a monovalent or polyvalent hydrocarbon moiety.

As used herein, "olefin" or "olefins" refer to compounds having at least one unsaturated carbon-carbon double bond. In certain embodiments, the term "olefins" refers to a group of unsaturated carbon-carbon double bond compounds with different carbon lengths. Unless noted otherwise, the terms "olefin" or "olefins" encompasses "polyunsaturated olefins" or "poly-olefins," which have more than one carbon-carbon double bond. As used herein, the term "monounsaturated olefins" or "mono-olefins" refers to compounds having only one carbon-carbon double bond. A compound having a terminal carbon-carbon double bond can be referred to as a "terminal olefin" or an "alpha-olefin," while an olefin having a non-terminal carbon-carbon double bond can be referred to as an "internal olefin." In some embodiments, the alpha-olefin is a terminal alkene, which is an alkene (as defined below) having a terminal carbon-carbon double bond. Additional carbon-carbon double bonds can be present.

The number of carbon atoms in any group or compound can be represented by the terms: "C_{z}", which refers to a group of compound having z carbon atoms; and "C_{x-y}", which refers to a group or compound containing from x to y, inclusive, carbon atoms. For example, "C₁₋₆ alkyl" represents an alkyl chain having from 1 to 6 carbon atoms and, for example, includes, but is not limited to, methyl, ethyl, n-propyl, isopropyl, isobutyl, n-butyl, sec-butyl, tert-butyl, isopentyl, n-pentyl, neopentyl, and n-hexyl. As a further example, a "C₄₋₁₀ alkene" refers to an alkene molecule having from 4 to 10 carbon atoms, and, for example, includes, but is not limited to, 1-butene, 2-butene, isobutene, 1-pentene, 1-hexene, 3-hexene, 1-heptene, 3-heptene, 1-octene, 4-octene, 1-nonene, 4-nonene, and 1-decene.

As used herein, the term "low-molecular-weight olefin" may refer to any one or combination of unsaturated straight, branched, or cyclic hydrocarbons in the C₂₋₁₄ range. Low-molecular-weight olefins include alpha-olefins, wherein the unsaturated carbon-carbon bond is present at one end of the compound. Low-molecular-weight olefins may also include dienes or trienes. Low-molecular-weight olefins may also include internal olefins or "low-molecular-weight internal olefins." In certain embodiments, the low-molecular-weight internal olefin is in the C₄₋₁₄ range. Examples of low-molecular-weight olefins in the C₂₋₆ range include, but are not limited to: ethylene, propylene, 1-butene, 2-butene, isobutene, 1-pentene, 2-pentene, 3-pentene, 2-methyl-1-butene, 2-methyl-2-butene, 3-methyl-1-butene, cyclopentene, 1,4-pentadiene, 1-hexene, 2-hexene, 3-hexene, 4-hexene, 2-methyl-1-pentene, 3-methyl-1-pentene, 4-methyl-1-pentene, 2-methyl-2-pentene, 3-methyl-2-pentene, 4-methyl-2-pentene, 2-methyl-3-pentene, and cyclohexene. Non-limiting examples of low-molecular-weight olefins in the C₇₋₉ range include 1,4-heptadiene, 1-heptene, 3,6-nonadiene, 3-nonene, 1,4,7-octatriene. Other possible low-molecular-weight olefins include styrene and vinyl cyclohexane. In certain embodiments, it is preferable to use a mixture of olefins, the mixture comprising linear and branched low-molecular-weight olefins in the C₄₋₁₀ range. Olefins in the C₄₋₁₀ range can also be referred to as "short-chain olefins," which can be either branched or unbranched. In one embodiments, it may be preferable to use a mixture of linear and branched C₄ olefins (i.e., combinations of: 1-butene, 2-butene, and/or isobutene). In other embodiments, a higher range of C₁₁₋₁₄ may be used.

In some instances, the olefin can be an "alkene," which refers to a straight- or branched-chain non-aromatic hydrocarbon having 2 to 30 carbon atoms and one or more carbon-carbon double bonds, which may be optionally substituted, as herein further described, with multiple degrees of substitution being allowed. A "monounsaturated alkene" refers to an alkene having one carbon-carbon double bond, while a "polyunsaturated alkene" refers to an alkene having two or more carbon-carbon double bonds. A "lower alkene," as used herein, refers to an alkene having from 2 to 10 carbon atoms.

As used herein, "ester" or "esters" refer to compounds having the general formula: R-COO-R', wherein R and R' denote any organic group (such as alkyl, aryl, or silyl groups) including those bearing heteroatom-containing substituent groups. In certain embodiments, R and R' denote alkyl, alkenyl, aryl, or alcohol groups. In certain embodiments, the term "esters" may refer to a group of compounds with the general formula described above, wherein the compounds have different carbon lengths. In certain embodiments, the esters may be esters of glycerol, which is a trihydric alcohol. The term "glyceride" can refer to esters where one, two, or three of the -OH groups of the glycerol have been esterified.

It is noted that an olefin may also comprise an ester, and an ester may also comprise an olefin, if the R or R' group in the general formula R-COO-R' contains an unsaturated carbon-carbon double bond. Such compounds can be referred to as "unsaturated esters" or "olefin ester" or "olefinic ester compounds." Further, a "terminal olefinic ester compound" may refer to an ester compound where R has an olefin positioned at the end of the chain. An "internal olefin ester" may refer to an ester compound where R has an olefin positioned at an internal location on the chain. Additionally, the term "terminal olefin" may refer to an ester or an acid thereof where R' denotes hydrogen or any organic compound (such as an alkyl, aryl, or silyl group) and R has an olefin positioned at the end of the chain, and the term "internal olefin" may refer to an ester or an acid thereof where R' denotes hydrogen or any organic compound (such as an alkyl, aryl, or silyl group) and R has an olefin positioned at an internal location on the chain.

As used herein, "alkyl" refers to a straight or branched chain saturated hydrocarbon having 1 to 30 carbon atoms, which may be optionally substituted, as herein further described, with multiple degrees of substitution being allowed. Examples of "alkyl," as used herein, include, but are not limited to, methyl, ethyl, n-propyl, isopropyl, isobutyl, n-butyl, sec-butyl, tert-butyl, isopentyl, n-pentyl, neopentyl, n-hexyl, and 2-ethylhexyl. The number of carbon atoms in an alkyl group is represented by the phrase "C_{x-y} alkyl," which refers to an alkyl group, as herein defined, containing from x to y, inclusive, carbon atoms. Thus, "C₁₋₆ alkyl" represents an alkyl chain having from 1 to 6 carbon atoms and, for example, includes, but is not limited to, methyl, ethyl, n-propyl, isopropyl, isobutyl, n-butyl, sec-butyl, tert-butyl, isopentyl, n-pentyl, neopentyl, and n-hexyl. In some instances, the "alkyl" group can be divalent, in which case the group can alternatively be referred to as an "alkylene" group. Also, in some instances, one or more of the carbon atoms in the alkyl or alkylene group can be replaced by a heteroatom (e.g., selected from nitrogen, oxygen, or sulfur, including N-oxides, sulfur oxides, and sulfur dioxides, where feasible), and is referred to as a "heteroalkyl" or "heteroalkylene" group, respectively. Non-limiting examples include "oxyalkyl" or "oxyalkylene" groups, which are groups of the following formulas: -[-(alkylene)-O-]ₓ-alkyl, or -[-(alkylene)-O-]ₓ-alkylene-, respectively, where x is 1 or more, such as 1, 2, 3, 4, 5, 6, 7, or 8.

As used herein, "alkenyl" refers to a straight or branched chain non-aromatic hydrocarbon having 2 to 30 carbon atoms and having one or more carbon-carbon double bonds, which may be optionally substituted, as herein further described, with multiple degrees of substitution being allowed. Examples of "alkenyl," as used herein, include, but are not limited to, ethenyl, 2-propenyl, 2-butenyl, and 3-butenyl. The number of carbon atoms in an alkenyl group is represented by the phrase "C_{x-y} alkenyl," which refers to an alkenyl group, as herein defined, containing from x to y, inclusive, carbon atoms. Thus, "C₂₋₆ alkenyl" represents an alkenyl chain having from 2 to 6 carbon atoms and, for example, includes, but is not limited to, ethenyl, 2-propenyl, 2-butenyl, and 3-butenyl. In some instances, the "alkenyl" group can be divalent, in which case the group can alternatively be referred to as an "alkenylene" group. Also, in some instances, one or more of the saturated carbon atoms in the alkenyl or alkenylene group can be replaced by a heteroatom (e.g., selected from nitrogen, oxygen, or sulfur, including N-oxides, sulfur oxides, and sulfur dioxides, where feasible), and is referred to as a "heteroalkenyl" or "heteroalkenylene" group, respectively. Non-limiting examples include "oxyalkenyl" or "oxyalkenylene" groups, which are groups of the following formulas: -[-(R^{f})-O-]ₓ-R^{g}, or -[-(R^{f})-O-]ₓ-R^{h}-, respectively, where x is 1 or more, such as 1, 2, 3, 4, 5, 6, 7, or 8, and R^{f}, R^{g}, and R^{h} are independently alkyl/alkylene or alkenyl/alkenylene groups, provided that each such "oxyalkenyl" or "oxyalkenylene" group contains at least one carbon-carbon double bond.

As used herein, "cycloalkyl" refers to an aliphatic saturated or unsaturated hydrocarbon ring system having 3 to 30 carbon atoms, which may be optionally substituted, as herein further described, with multiple degrees of substitution being allowed. Examples of "cycloalkyl," as used herein, include, but are not limited to, cyclopropyl, cyclobutyl, cyclopentyl, cyclohexyl, cyclohexenyl, cycloheptyl, cyclooctyl, adamantyl, and the like. The number of carbon atoms in a cycloalkyl group is represented by the phrase "C_{x-y} cycloalkyl," which refers to a cycloalkyl group, as herein defined, containing from x to y, inclusive, carbon atoms. Thus, "C₃₋₁₀ cycloalkyl" represents a cycloalkyl having from 3 to 10 carbon atoms and, for example, includes, but is not limited to, cyclopropyl, cyclobutyl, cyclopentyl, cyclohexyl, cyclohexenyl, cycloheptyl, cyclooctyl, and adamantyl. In some instances, the "cycloalkyl" group can be divalent, in which case the group can alternatively be referred to as a "cycloalkylene" group. Also, in some instances, one or more of the carbon atoms in the cycloalkyl or cycloalkylene group can be replaced by a heteroatom (e.g., selected from nitrogen, oxygen, or sulfur, including N-oxides, sulfur oxides, and sulfur dioxides, where feasible), and is referred to as a "heterocycloalkyl" or "heterocycloalkylene" group, respectively.

As used herein, "halogen" or "halo" refers to a fluorine, chlorine, bromine, and/or iodine atom. In some embodiments, the terms refer to fluorine and/or chlorine.

As used herein, "substituted" refers to substitution of one or more hydrogen atoms of the designated moiety with the named substituent or substituents, multiple degrees of substitution being allowed unless otherwise stated, provided that the substitution results in a stable or chemically feasible compound. A stable compound or chemically feasible compound is one in which the chemical structure is not substantially altered when kept at a temperature from about -80 °C to about +40 °C, in the absence of moisture or other chemically reactive conditions, for at least a week. As used herein, the phrases "substituted with one or more..." or "substituted one or more times..." refer to a number of substituents that equals from one to the maximum number of substituents possible based on the number of available bonding sites, provided that the above conditions of stability and chemical feasibility are met.

As used herein, "yield" refers to the amount of reaction product formed in a reaction. When expressed with units of percent (%), the term yield refers to the amount of reaction product actually formed, as a percentage of the amount of reaction product that would be formed if all of the limiting reactant were converted into the product.

As used herein, "mix" or "mixed" or "mixture" refers broadly to any combining of two or more compositions. The two or more compositions need not have the same physical state; thus, solids can be "mixed" with liquids, e.g., to form a slurry, suspension, or solution. Further, these terms do not require any degree of homogeneity or uniformity of composition. This, such "mixtures" can be homogeneous or heterogeneous, or can be uniform or non-uniform. Further, the terms do not require the use of any particular equipment to carry out the mixing, such as an industrial mixer.

As used herein, "optionally" means that the subsequently described event(s) may or may not occur. In some embodiments, the optional event does not occur. In some other embodiments, the optional event does occur one or more times.

As used herein, "comprise" or "comprises" or "comprising" or "comprised of" refer to groups that are open, meaning that the group can include additional members in addition to those expressly recited. For example, the phrase, "comprises A" means that A must be present, but that other members can be present too. The terms "include," "have," and "composed of" and their grammatical variants have the same meaning. In contrast, "consist of" or "consists of" or "consisting of" refer to groups that are closed. For example, the phrase "consists of A" means that A and only A is present.

As used herein, "or" is to be given its broadest reasonable interpretation, and is not to be limited to an either/or construction. Thus, the phrase "comprising A or B" means that A can be present and not B, or that B is present and not A, or that A and B are both present. Further, if A, for example, defines a class that can have multiple members, e.g., A₁ and A₂, then one or more members of the class can be present concurrently.

As used herein, the various functional groups represented will be understood to have a point of attachment at the functional group having the hyphen or dash (-) or an asterisk (*). In other words, in the case of -CH₂CH₂CH₃, it will be understood that the point of attachment is the CH₂ group at the far left. If a group is recited without an asterisk or a dash, then the attachment point is indicated by the plain and ordinary meaning of the recited group.

As used herein, multi-atom bivalent species are to be read from left to right. For example, if the specification or claims recite A-D-E and D is defined as -OC(O)-, the resulting group with D replaced is: A-OC(O)-E and not A-C(O)O-E.

Other terms are defined in other portions of this description, even though not included in this subsection.

### Branched-Chain Ester Compounds

In certain aspects, the disclosure provides branched-chain monoesters. In some embodiments, the branched-chain monoesters have an estolide-like linkage, e.g., where branching occurs at the carbon immediately adjacent to the alcoholic oxygen of the ester group. In some embodiments, the branched-chain esters are compounds of Formula (I): 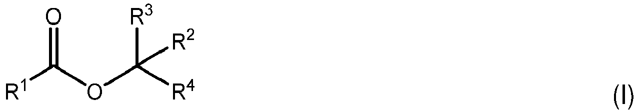 wherein: R¹ is C₃₋₂₄ alkyl or C₃₋₂₄ alkenyl, each of which is optionally substituted one or more times with substituents selected from R⁵; R² is a hydrogen atom or C₁₋₆ alkyl, which is optionally substituted one or more times with substituents selected from R⁵; and R³ and R⁴ are independently C₃₋₂₄ alkyl or C₃₋₂₄ alkenyl, each of which is optionally substituted one or more times with substituents selected from R⁵; and R⁵ is a halogen atom, -OH, -NH₂, C₁₋₆ alkyl, C₁₋₆ heteroalkyl, C₂₋₆ alkenyl, C2-6 heteroalkenyl, C₃₋₁₀ cyclokalkyl, or C₂₋₁₀ heterocycloalkyl.

In some embodiments, R¹ is C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₃₋₁₄ alkyl or C₃₋₁₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₅₋₁₂ alkyl or C₅₋₁₂ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.

In some embodiments, R¹ is C₃₋₂₄ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₃₋₁₄ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₅₋₁₂ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.

In some embodiments, R¹ is C₃₋₂₄ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₃₋₁₄ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R¹ is C₅₋₁₂ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some such embodiments, the alkenyl group is a terminal alkenyl group.

In some embodiments, R¹ is C₃₋₂₄ alkyl or C₃₋₂₄ alkenyl, each of which can be optionally substituted one or more times with -OH. In some embodiments, R¹ is C₃₋₁₄ alkyl or C₃₋₁₄ alkenyl, each of which can be optionally substituted one or more times with -OH. In some embodiments, R¹ is C₅₋₁₂ alkyl or C₅₋₁₂ alkenyl, each of which can be optionally substituted one or more times with -OH.

In some embodiments, R¹ is C₃₋₂₄ alkyl, which can be optionally substituted one or more times with -OH. In some embodiments, R¹ is C₃₋₁₄ alkyl, which can be optionally substituted one or more times with -OH. In some embodiments, R¹ is C₅₋₁₂ alkyl, which can be optionally substituted one or more times with -OH.

In some embodiments, R¹ is C₃₋₂₄ alkenyl, which can be optionally substituted one or more times with -OH. In some embodiments, R¹ is C₃₋₁₄ alkenyl, which can be optionally substituted one or more times with -OH. In some embodiments, R¹ is C₅₋₁₂ alkenyl, which can be optionally substituted one or more times with -OH. In some such embodiments, the alkenyl group is a terminal alkenyl group.

In some embodiments, R¹ is pentyl, hexyl, heptyl, octyl, nonyl, decyl, undecyl, dodecyl, tridecyl, tetradecyl, pentadecyl, hexadecyl, or heptadecyl. In some embodiments, R¹ is nonyl, decyl, or undecyl. In some embodiments, R¹ is nonyl or undecyl. In some embodiments, R¹ is nonyl. In some other embodiments, R¹ is 8-nonenyl, 8-decenyl, or 8-undecenyl. In some other embodiments, R¹ is 8-nonenyl or 8-undecenyl. In some embodiments, R¹ is 8-nonenyl.

In some embodiments, R² is methyl of a hydrogen atom. In some embodiments, R² is a hydrogen atom.

In some embodiments, R³ and R⁴ are independently C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₃₋₁₄ alkyl or C₃₋₁₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₅₋₁₂ alkyl or C₅₋₁₂ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.

In some embodiments, R³ and R⁴ are independently C₃₋₂₄ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₃₋₁₄ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₅₋₁₂ alkyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.

In some embodiments, R³ and R⁴ are independently C₃₋₂₄ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₃₋₁₄ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some embodiments, R³ and R⁴ are independently C₅₋₁₂ alkenyl, which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂. In some such embodiments, the alkenyl group is a terminal alkenyl group.

In some embodiments, R³ and R⁴ are independently C₃₋₂₄ alkyl or C₃₋₂₄ alkenyl, each of which can be optionally substituted one or more times with -OH. In some embodiments, R³ and R⁴ are independently C₃₋₁₄ alkyl or C₃₋₁₄ alkenyl, each of which can be optionally substituted one or more times with -OH. In some embodiments, R³ and R⁴ are independently C₅₋₁₂ alkyl or C₅₋₁₂ alkenyl, each of which can be optionally substituted one or more times with -OH.

In some embodiments, R³ and R⁴ are independently C₃₋₂₄ alkyl, which can be optionally substituted one or more times with -OH. In some embodiments, R³ and R⁴ are independently C₃₋₁₄ alkyl, which can be optionally substituted one or more times with -OH. In some embodiments, R³ and R⁴ are independently C₅₋₁₂ alkyl, which can be optionally substituted one or more times with -OH.

In some embodiments, R³ and R⁴ are independently C₃₋₂₄ alkenyl, which can be optionally substituted one or more times with -OH. In some embodiments, R³ and R⁴ are independently C₃₋₁₄ alkenyl, which can be optionally substituted one or more times with -OH. In some embodiments, R³ and R⁴ are independently C₅₋₁₂ alkenyl, which can be optionally substituted one or more times with -OH. In some such embodiments, the alkenyl group is a terminal alkenyl group.

In some embodiments, R³ and R⁴ are independently propyl, butyl, pentyl, hexyl, heptyl, octyl, nonyl, decyl, undecyl, dodecyl, tridecyl, tetradecyl, pentadecyl, hexadecyl, or heptadecyl. In some embodiments, R³ and R⁴ are independently propyl, butyl, pentyl, hexyl, heptyl, octyl, nonyl, or decyl. In some embodiments, at least one of R³ and R⁴ is octyl or nonyl. In some embodiments, one of R³ and R⁴ is octyl, and the other is nonyl.

In some embodiments, at least one of R³ or R⁴ can be branched, e.g., a branched alkyl group being substituted in a manner consistent with any of the above embodiments. In some embodiments, at least one of R³ or R⁴ is -CH(CH₃)-(CH₂)₃-CH₃, -CH(CH₃)-(CH₂)₄-CH₃, -CH(CH₃)-(CH₂)₅-CH₃, -CH(CH3)-(CH2)6-CH3, -CH(CH3)-(CH2)7-CH3, -CH(CH3)-(CH2)8-CH3, or -CH(CH₃)-(CH₂)₉-CH₃, while the other is a group according to any of the above embodiments. In some other embodiments, at least one of R³ or R⁴ is -CH₂-CH(CH₃)-(CH₂)₂-CH₃, -CH₂-CH(CH₃)-(CH₂)₃-CH₃, -CH₂-CH(CH₃)-(CH₂)₄-CH₃, -CH₂-CH(CH₃)-(CH₂)₅-CH₃, -CH₂-CH(CH₃)-(CH₂)₆-CH₃, -CH₂-CH(CH₃)-(CH₂)₇-CH₃, or -CH₂-CH(CH₃)-(CH₂)₈-CH₃, while the other is a group according to any of the above embodiments. In some embodiments, one of R³ or R⁴ is -CH[-(CH₂)₇-CH₃][-CH₂-CH(CH₃)-(CH2)₇-CH₃], while the other is a group according to any of the above embodiments, such as octyl or nonyl.

Branched ester compounds of this disclosure can be made in any suitable manner. In some embodiments, an internal olefin (e.g., a C₆₋₄₈ olefin) is reacted at one of its carbon-carbon double bonds with a carboxylic acid through a condensation reaction. In some embodiments, for example, an internal olefin is reacted with a carboxylic acid at an elevated temperature (e.g., 35-100 °C), optionally in the presence of another acid, such as a superacid. In at least one example, 9-octadecene is reacted with decanoic acid at about 55 °C in the presence of an acid catalyst to form a composition predominantly containing 1-octyldecyl decanoate. In some embodiments, synthesis methods analogous to those described in United States Patent Nos. 8,450,256, 8,455,412, 8,486, 875, and 8,637,689, and in United States Patent Application Publication Nos. 2013/0245298 and 2013/0274493, all of which are incorporated by reference as though fully set forth herein.

As noted above, the branched-chain esters disclosed herein can be made from the reaction of a carboxylic acid with an olefin. Any suitable olefins can be used, including, but not limited to, 1-decene, 1,4-decadiene, 3-dodecene, 6-dodecene, 3,6-dodecadiene, 1,4-tridecadiene, 6-pentadecene, 3,6-pentadecadiene, and 9-octadecene. In some embodiments, the dienes may be partially hydrogenated, leading to 4-decene, 1-tridecene, 4-tridecene, and 3-pentadecene. In some embodiments, the olefins can be further isomerized to form other olefins. Further, any of the above olefins can be further reacted with each other, e.g., by metathesis, to form longer-chain olefins. For example, in some embodiments, additional 9-octadecene may be made via the self-metathesis of 1-decene or 3-dodecene, or via the cross-metathesis of 1-decene with 3-dodecene. Any suitable acids can be used, including, but not limited to, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, and stearic acid. Unsaturated acids can also be used, such as 9-decenoic acid, 3-dodecenoic acid, 9-pentadecenoic acid, oleic acid, and the like. Such unsaturated acids can also be chain-lengthened or chain-shortened, e.g., via metathesis, to make unsaturated acids having different chain lengths.

### Derivation from Renewable Sources

The branched ester compounds employed in any of the aspects or embodiments disclosed herein can, in certain embodiments, be derived from renewable sources, such as from various natural oils or their derivatives. Any suitable methods can be used to make these compounds from such renewable sources. Suitable methods include, but are not limited to, fermentation, conversion by bioorganisms, and conversion by metathesis.

Olefin metathesis provides one possible means to convert certain natural oil feedstocks into olefins and esters that can be used in a variety of applications, or that can be further modified chemically and used in a variety of applications. In some embodiments, a composition (or components of a composition) may be formed from a renewable feedstock, such as a renewable feedstock formed through metathesis reactions of natural oils and/or their fatty acid or fatty ester derivatives. When compounds containing a carbon-carbon double bond undergo metathesis reactions in the presence of a metathesis catalyst, some or all of the original carbon-carbon double bonds are broken, and new carbon-carbon double bonds are formed. The products of such metathesis reactions include carbon-carbon double bonds in different locations, which can provide unsaturated organic compounds having useful chemical properties.

A wide range of natural oils, or derivatives thereof, can be used in such metathesis reactions. Examples of suitable natural oils include, but are not limited to, vegetable oils, algae oils, fish oils, animal fats, tall oils, derivatives of these oils, combinations of any of these oils, and the like. Representative non-limiting examples of vegetable oils include rapeseed oil (canola oil), coconut oil, corn oil, cottonseed oil, olive oil, palm oil, peanut oil, safflower oil, sesame oil, soybean oil, sunflower oil, linseed oil, palm kernel oil, tung oil, jatropha oil, mustard seed oil, pennycress oil, camelina oil, hempseed oil, and castor oil. Representative non-limiting examples of animal fats include lard, tallow, poultry fat, yellow grease, and fish oil. Tall oils are by-products of wood pulp manufacture. In some embodiments, the natural oil or natural oil feedstock comprises one or more unsaturated glycerides (e.g., unsaturated triglycerides). In some such embodiments, the natural oil feedstock comprises at least 50% by weight, or at least 60% by weight, or at least 70% by weight, or at least 80% by weight, or at least 90% by weight, or at least 95% by weight, or at least 97% by weight, or at least 99% by weight of one or more unsaturated triglycerides, based on the total weight of the natural oil feedstock.

The natural oil may include canola or soybean oil, such as refined, bleached and deodorized soybean oil (i.e., RBD soybean oil). Soybean oil typically includes about 95 percent by weight (wt%) or greater (e.g., 99 wt% or greater) triglycerides of fatty acids. Major fatty acids in the polyol esters of soybean oil include but are not limited to saturated fatty acids such as palmitic acid (hexadecanoic acid) and stearic acid (octadecanoic acid), and unsaturated fatty acids such as oleic acid (9-octadecenoic acid), linoleic acid (9,12-octadecadienoic acid), and linolenic acid (9,12,15-octadecatrienoic acid).

Metathesized natural oils can also be used. Examples of metathesized natural oils include but are not limited to a metathesized vegetable oil, a metathesized algal oil, a metathesized animal fat, a metathesized tall oil, a metathesized derivatives of these oils, or mixtures thereof. For example, a metathesized vegetable oil may include metathesized canola oil, metathesized rapeseed oil, metathesized coconut oil, metathesized corn oil, metathesized cottonseed oil, metathesized olive oil, metathesized palm oil, metathesized peanut oil, metathesized safflower oil, metathesized sesame oil, metathesized soybean oil, metathesized sunflower oil, metathesized linseed oil, metathesized palm kernel oil, metathesized tung oil, metathesized jatropha oil, metathesized mustard oil, metathesized camelina oil, metathesized pennycress oil, metathesized castor oil, metathesized derivatives of these oils, or mixtures thereof. In another example, the metathesized natural oil may include a metathesized animal fat, such as metathesized lard, metathesized tallow, metathesized poultry fat, metathesized fish oil, metathesized derivatives of these oils, or mixtures thereof.

Such natural oils, or derivatives thereof, can contain esters, such as triglycerides, of various unsaturated fatty acids. The identity and concentration of such fatty acids varies depending on the oil source, and, in some cases, on the variety. In some embodiments, the natural oil comprises one or more esters of oleic acid, linoleic acid, linolenic acid, or any combination thereof. When such fatty acid esters are metathesized, new compounds are formed. For example, in embodiments where the metathesis uses certain short-chain olefins, e.g., ethylene, propylene, or 1-butene, and where the natural oil includes esters of oleic acid, an amount of 1-decene and 1-decenoid acid (or an ester thereof), among other products, are formed. Following transesterification, for example, with an alkyl alcohol, an amount of 9-denenoic acid alkyl ester is formed. In some such embodiments, a separation step may occur between the metathesis and the transesterification, where the alkenes are separated from the esters. In some other embodiments, transesterification can occur before metathesis, and the metathesis is performed on the transesterified product.

In some embodiments, the natural oil can be subjected to various pre-treatment processes, which can facilitate their utility for use in certain metathesis reactions. Useful pre-treatment methods are described in United States Patent Application Publication Nos. 2011/0113679, 2014/0275595, and 2014/0275681, all three of which are hereby incorporated by reference as though fully set forth herein.

In some embodiments, after any optional pre-treatment of the natural oil feedstock, the natural oil feedstock is reacted in the presence of a metathesis catalyst in a metathesis reactor. In some other embodiments, an unsaturated ester (e.g., an unsaturated glyceride, such as an unsaturated triglyceride) is reacted in the presence of a metathesis catalyst in a metathesis reactor. These unsaturated esters may be a component of a natural oil feedstock, or may be derived from other sources, e.g., from esters generated in earlier-performed metathesis reactions. In certain embodiments, in the presence of a metathesis catalyst, the natural oil or unsaturated ester can undergo a self-metathesis reaction with itself. In other embodiments, the natural oil or unsaturated ester undergoes a cross-metathesis reaction with the low-molecular-weight olefin or mid-weight olefin. The self-metathesis and/or cross-metathesis reactions form a metathesized product wherein the metathesized product comprises olefins and esters.

In some embodiments, the low-molecular-weight olefin (or short-chain olefin) is in the C₂₋₆ range. As a non-limiting example, in one embodiment, the low-molecular-weight olefin may comprise at least one of: ethylene, propylene, 1-butene, 2-butene, isobutene, 1-pentene, 2-pentene, 3-pentene, 2-methyl-1-butene, 2-methyl-2-butene, 3-methyl-1-butene, cyclopentene, 1,4-pentadiene, 1-hexene, 2-hexene, 3-hexene, 4-hexene, 2-methyl-1-pentene, 3-methyl-1-pentene, 4-methyl-1-pentene, 2-methyl-2-pentene, 3-methyl-2-pentene, 4-methyl-2-pentene, 2-methyl-3-pentene, and cyclohexene. In some embodiments, the short-chain olefin is 1-butene. In some instances, a higher-molecular-weight olefin can also be used.

In some embodiments, the metathesis comprises reacting a natural oil feedstock (or another unsaturated ester) in the presence of a metathesis catalyst. In some such embodiments, the metathesis comprises reacting one or more unsaturated glycerides (e.g., unsaturated triglycerides) in the natural oil feedstock in the presence of a metathesis catalyst. In some embodiments, the unsaturated glyceride comprises one or more esters of oleic acid, linoleic acid, linoleic acid, or combinations thereof. In some other embodiments, the unsaturated glyceride is the product of the partial hydrogenation and/or the metathesis of another unsaturated glyceride (as described above). In some such embodiments, the metathesis is a cross-metathesis of any of the aforementioned unsaturated triglyceride species with another olefin, e.g., an alkene. In some such embodiments, the alkene used in the cross-metathesis is a lower alkene, such as ethylene, propylene, 1-butene, 2-butene, etc. In some embodiments, the alkene is ethylene. In some other embodiments, the alkene is propylene. In some further embodiments, the alkene is 1-butene. And in some even further embodiments, the alkene is 2-butene.

Metathesis reactions can provide a variety of useful products, when employed in the methods disclosed herein. For example, the unsaturated esters may be derived from a natural oil feedstock, in addition to other valuable compositions. Moreover, in some embodiments, a number of valuable compositions can be targeted through the self-metathesis reaction of a natural oil feedstock, or the cross-metathesis reaction of the natural oil feedstock with a low-molecular-weight olefin or mid-weight olefin, in the presence of a metathesis catalyst. Such valuable compositions can include fuel compositions, detergents, surfactants, and other specialty chemicals. Additionally, transesterified products (i.e., the products formed from transesterifying an ester in the presence of an alcohol) may also be targeted, non-limiting examples of which include: fatty acid methyl esters ("FAMEs"); biodiesel; 9-decenoic acid ("9DA") esters, 9-undecenoic acid ("9UDA") esters, and/or 9-dodecenoic acid ("9DDA") esters; 9DA, 9UDA, and/or 9DDA; alkali metal salts and alkaline earth metal salts of 9DA, 9UDA, and/or 9DDA; dimers of the transesterified products; and mixtures thereof.

Further, in some embodiments, multiple metathesis reactions can also be employed. In some embodiments, the multiple metathesis reactions occur sequentially in the same reactor. For example, a glyceride containing linoleic acid can be metathesized with a terminal lower alkene (e.g., ethylene, propylene, 1-butene, and the like) to form 1,4-decadiene, which can be metathesized a second time with a terminal lower alkene to form 1,4-pentadiene. In other embodiments, however, the multiple metathesis reactions are not sequential, such that at least one other step (e.g., transesterification, hydrogenation, etc.) can be performed between the first metathesis step and the following metathesis step. These multiple metathesis procedures can be used to obtain products that may not be readily obtainable from a single metathesis reaction using available starting materials. For example, in some embodiments, multiple metathesis can involve self-metathesis followed by cross-metathesis to obtain metathesis dimers, trimmers, and the like. In some other embodiments, multiple metathesis can be used to obtain olefin and/or ester components that have chain lengths that may not be achievable from a single metathesis reaction with a natural oil triglyceride and typical lower alkenes (e.g., ethylene, propylene, 1-butene, 2-butene, and the like). Such multiple metathesis can be useful in an industrial-scale reactor, where it may be easier to perform multiple metathesis than to modify the reactor to use a different alkene.

For example, multiple metathesis can be employed to make the extended-chain branched-chain ester compounds disclosed herein. In some embodiments, cross-metathesis of an oleate can yield 1-decene, which can be self-metathesized to form 9-octadecene, which can react with via condensation with an acid to form a branched-chain ester. The ester portion of the branched ester can also be derived from a renewable source. For example, cross-metathesis of an oleate can also yield 9-decenoate, which can be hydrolyzed to 9-decenoic acid, which can be hydrogenated to form decanoic acid. Other branched-chain ester compounds can be derived from renewable sources by analogous means.

The conditions for such metathesis reactions, and the reactor design, and suitable catalysts are as described below with reference to the metathesis of the olefin esters. That discussion is incorporated by reference as though fully set forth herein.

In the embodiments above, the natural oil (e.g., as a glyceride) is metathesized, followed by transesterification. In some other embodiments, transesterification can precede metathesis, such that the fatty acid esters subjected to metathesis are fatty acid esters of monohydric alcohols, such as methanol, ethanol, or isopropanol.

### Olefin Metathesis

In some embodiments, one or more of the unsaturated monomers can be made by metathesizing a natural oil or natural oil derivative. The terms "metathesis" or "metathesizing" can refer to a variety of different reactions, including, but not limited to, cross-metathesis, self-metathesis, ring-opening metathesis, ring-opening metathesis polymerizations ("ROMP"), ring-closing metathesis ("RCM"), and acyclic diene metathesis ("ADMET"). Any suitable metathesis reaction can be used, depending on the desired product or product mixture.

In some embodiments, after any optional pre-treatment of the natural oil feedstock, the natural oil feedstock is reacted in the presence of a metathesis catalyst in a metathesis reactor. In some other embodiments, an unsaturated ester (e.g., an unsaturated glyceride, such as an unsaturated triglyceride) is reacted in the presence of a metathesis catalyst in a metathesis reactor. These unsaturated esters may be a component of a natural oil feedstock, or may be derived from other sources, e.g., from esters generated in earlier-performed metathesis reactions. In certain embodiments, in the presence of a metathesis catalyst, the natural oil or unsaturated ester can undergo a self-metathesis reaction with itself. In other embodiments, the natural oil or unsaturated ester undergoes a cross-metathesis reaction with the low-molecular-weight olefin or mid-weight olefin. The self-metathesis and/or cross-metathesis reactions form a metathesized product wherein the metathesized product comprises olefins and esters.

In some embodiments, the low-molecular-weight olefin is in the C₂₋₆ range. As a non-limiting example, in one embodiment, the low-molecular-weight olefin may comprise at least one of: ethylene, propylene, 1-butene, 2-butene, isobutene, 1-pentene, 2-pentene, 3-pentene, 2-methyl-1-butene, 2-methyl-2-butene, 3-methyl-1-butene, cyclopentene, 1,4-pentadiene, 1-hexene, 2-hexene, 3-hexene, 4-hexene, 2-methyl-1-pentene, 3-methyl-1-pentene, 4-methyl-1-pentene, 2-methyl-2-pentene, 3-methyl-2-pentene, 4-methyl-2-pentene, 2-methyl-3-pentene, and cyclohexene. In some instances, a higher-molecular-weight olefin can also be used.

In some embodiments, the metathesis comprises reacting a natural oil feedstock (or another unsaturated ester) in the presence of a metathesis catalyst. In some such embodiments, the metathesis comprises reacting one or more unsaturated glycerides (e.g., unsaturated triglycerides) in the natural oil feedstock in the presence of a metathesis catalyst. In some embodiments, the unsaturated glyceride comprises one or more esters of oleic acid, linoleic acid, linoleic acid, or combinations thereof. In some other embodiments, the unsaturated glyceride is the product of the partial hydrogenation and/or the metathesis of another unsaturated glyceride (as described above). In some such embodiments, the metathesis is a cross-metathesis of any of the aforementioned unsaturated triglyceride species with another olefin, e.g., an alkene. In some such embodiments, the alkene used in the cross-metathesis is a lower alkene, such as ethylene, propylene, 1-butene, 2-butene, etc. In some embodiments, the alkene is ethylene. In some other embodiments, the alkene is propylene. In some further embodiments, the alkene is 1-butene. And in some even further embodiments, the alkene is 2-butene.

Metathesis reactions can provide a variety of useful products, when employed in the methods disclosed herein. For example, terminal olefins and internal olefins may be derived from a natural oil feedstock, in addition to other valuable compositions. Moreover, in some embodiments, a number of valuable compositions can be targeted through the self-metathesis reaction of a natural oil feedstock, or the cross-metathesis reaction of the natural oil feedstock with a low-molecular-weight olefin or mid-weight olefin, in the presence of a metathesis catalyst. Such valuable compositions can include fuel compositions, detergents, surfactants, and other specialty chemicals. Additionally, transesterified products (i.e., the products formed from transesterifying an ester in the presence of an alcohol) may also be targeted, non-limiting examples of which include: fatty acid methyl esters ("FAMEs"); biodiesel; 9-decenoic acid ("9DA") esters, 9-undecenoic acid ("9UDA") esters, and/or 9-dodecenoic acid ("9DDA") esters; 9DA, 9UDA, and/or 9DDA; alkali metal salts and alkaline earth metal salts of 9DA, 9UDA, and/or 9DDA; dimers of the transesterified products; and mixtures thereof.

Further, in some embodiments, the methods disclosed herein can employ multiple metathesis reactions. In some embodiments, the multiple metathesis reactions occur sequentially in the same reactor. For example, a glyceride containing linoleic acid can be metathesized with a terminal lower alkene (e.g., ethylene, propylene, 1-butene, and the like) to form 1,4-decadiene, which can be metathesized a second time with a terminal lower alkene to form 1,4-pentadiene. In other embodiments, however, the multiple metathesis reactions are not sequential, such that at least one other step (e.g., transesterification, hydrogenation, etc.) can be performed between the first metathesis step and the following metathesis step. These multiple metathesis procedures can be used to obtain products that may not be readily obtainable from a single metathesis reaction using available starting materials. For example, in some embodiments, multiple metathesis can involve self-metathesis followed by cross-metathesis to obtain metathesis dimers, trimers, and the like. In some other embodiments, multiple metathesis can be used to obtain olefin and/or ester components that have chain lengths that may not be achievable from a single metathesis reaction with a natural oil triglyceride and typical lower alkenes (e.g., ethylene, propylene, 1-butene, 2-butene, and the like). Such multiple metathesis can be useful in an industrial-scale reactor, where it may be easier to perform multiple metathesis than to modify the reactor to use a different alkene.

The metathesis process can be conducted under any conditions adequate to produce the desired metathesis products. For example, stoichiometry, atmosphere, solvent, temperature, and pressure can be selected by one skilled in the art to produce a desired product and to minimize undesirable byproducts. In some embodiments, the metathesis process may be conducted under an inert atmosphere. Similarly, in embodiments where a reagent is supplied as a gas, an inert gaseous diluent can be used in the gas stream. In such embodiments, the inert atmosphere or inert gaseous diluent typically is an inert gas, meaning that the gas does not interact with the metathesis catalyst to impede catalysis to a substantial degree. For example, non-limiting examples of inert gases include helium, neon, argon, and nitrogen, used individually or in with each other and other inert gases.

The rector design for the metathesis reaction can vary depending on a variety of factors, including, but not limited to, the scale of the reaction, the reaction conditions (heat, pressure, etc.), the identity of the catalyst, the identity of the materials being reacted in the reactor, and the nature of the feedstock being employed. Suitable reactors can be designed by those of skill in the art, depending on the relevant factors, and incorporated into a refining process such, such as those disclosed herein.

The metathesis reactions disclosed herein generally occur in the presence of one or more metathesis catalysts. Such methods can employ any suitable metathesis catalyst. The metathesis catalyst in this reaction may include any catalyst or catalyst system that catalyzes a metathesis reaction. Any known metathesis catalyst may be used, alone or in combination with one or more additional catalysts. Examples of metathesis catalysts and process conditions are described in US 2011/0160472, incorporated by reference herein in its entirety, except that in the event of any inconsistent disclosure or definition from the present specification, the disclosure or definition herein shall be deemed to prevail. A number of the metathesis catalysts described in US 2011/0160472 are presently available from Materia, Inc. (Pasadena, Calif.).

In some embodiments, the metathesis catalyst includes a Grubbs-type olefin metathesis catalyst and/or an entity derived therefrom. In some embodiments, the metathesis catalyst includes a first-generation Grubbs-type olefin metathesis catalyst and/or an entity derived therefrom. In some embodiments, the metathesis catalyst includes a second-generation Grubbs-type olefin metathesis catalyst and/or an entity derived therefrom. In some embodiments, the metathesis catalyst includes a first-generation Hoveyda-Grubbs-type olefin metathesis catalyst and/or an entity derived therefrom. In some embodiments, the metathesis catalyst includes a second-generation Hoveyda-Grubbs-type olefin metathesis catalyst and/or an entity derived therefrom. In some embodiments, the metathesis catalyst includes one or a plurality of the ruthenium carbene metathesis catalysts sold by Materia, Inc. of Pasadena, California and/or one or more entities derived from such catalysts. Representative metathesis catalysts from Materia, Inc. for use in accordance with the present teachings include but are not limited to those sold under the following product numbers as well as combinations thereof: product no. C823 (CAS no. 172222-30-9), product no. C848 (CAS no. 246047-72-3), product no. C601 (CAS no. 203714-71-0), product no. C627 (CAS no. 301224-40-8), product no. C571 (CAS no. 927429-61-6), product no. C598 (CAS no. 802912-44-3), product no. C793 (CAS no. 927429-60-5), product no. C801 (CAS no. 194659-03-9), product no. C827 (CAS no. 253688-91-4), product no. C884 (CAS no. 900169-53-1), product no. C833 (CAS no. 1020085-61-3), product no. C859 (CAS no. 832146-68-6), product no. C711 (CAS no. 635679-24-2), product no. C933 (CAS no. 373640-75-6).

In some embodiments, the metathesis catalyst includes a molybdenum and/or tungsten carbene complex and/or an entity derived from such a complex. In some embodiments, the metathesis catalyst includes a Schrock-type olefin metathesis catalyst and/or an entity derived therefrom. In some embodiments, the metathesis catalyst includes a high-oxidation-state alkylidene complex of molybdenum and/or an entity derived therefrom. In some embodiments, the metathesis catalyst includes a high-oxidation-state alkylidene complex of tungsten and/or an entity derived therefrom. In some embodiments, the metathesis catalyst includes molybdenum (VI). In some embodiments, the metathesis catalyst includes tungsten (VI). In some embodiments, the metathesis catalyst includes a molybdenum- and/or a tungsten-containing alkylidene complex of a type described in one or more of (a) Angew. Chem. Int. Ed. Engl., 2003, 42, 4592-4633; (b) Chem. Rev., 2002, 102, 145-179; and/or (c) Chem. Rev., 2009, 109, 3211-3226, each of which is incorporated by reference herein in its entirety, except that in the event of any inconsistent disclosure or definition from the present specification, the disclosure or definition herein shall be deemed to prevail.

In certain embodiments, the metathesis catalyst is dissolved in a solvent prior to conducting the metathesis reaction. In certain such embodiments, the solvent chosen may be selected to be substantially inert with respect to the metathesis catalyst. For example, substantially inert solvents include, without limitation: aromatic hydrocarbons, such as benzene, toluene, xylenes, etc.; halogenated aromatic hydrocarbons, such as chlorobenzene and dichlorobenzene; aliphatic solvents, including pentane, hexane, heptane, cyclohexane, etc.; and chlorinated alkanes, such as dichloromethane, chloroform, dichloroethane, etc. In some embodiments, the solvent comprises toluene.

In other embodiments, the metathesis catalyst is not dissolved in a solvent prior to conducting the metathesis reaction. The catalyst, instead, for example, can be slurried with the natural oil or unsaturated ester, where the natural oil or unsaturated ester is in a liquid state. Under these conditions, it is possible to eliminate the solvent (e.g., toluene) from the process and eliminate downstream olefin losses when separating the solvent. In other embodiments, the metathesis catalyst may be added in solid state form (and not slurried) to the natural oil or unsaturated ester (e.g., as an auger feed).

The metathesis reaction temperature may, in some instances, be a rate-controlling variable where the temperature is selected to provide a desired product at an acceptable rate. In certain embodiments, the metathesis reaction temperature is greater than -40 °C, or greater than -20 °C, or greater than 0 °C, or greater than 10 °C. In certain embodiments, the metathesis reaction temperature is less than 200 °C, or less than 150 °C, or less than 120 °C. In some embodiments, the metathesis reaction temperature is between 0 °C and 150 °C, or is between 10 °C and 120 °C.

The metathesis reaction can be run under any desired pressure. In some instances, it may be desirable to maintain a total pressure that is high enough to keep the cross-metathesis reagent in solution. Therefore, as the molecular weight of the cross-metathesis reagent increases, the lower pressure range typically decreases since the boiling point of the cross-metathesis reagent increases. The total pressure may be selected to be greater than 0.1 atm (10 kPa), or greater than 0.3 atm (30 kPa), or greater than 1 atm (100 kPa). In some embodiments, the reaction pressure is no more than about 70 atm (7000 kPa), or no more than about 30 atm (3000 kPa). In some embodiments, the pressure for the metathesis reaction ranges from about 1 atm (100 kPa) to about 30 atm (3000 kPa).

### Lubricant Compositions, and Methods of Making and Using the Same

In certain aspects, the disclosure provides lubricant compositions that include branched-chain ester compounds according to any of the above embodiments. In some embodiments, the lubricant compositions can contain one or more additional ingredients.

The disclosed branched-chain esters can be included in a lubricant composition with any other suitable ingredients. In some embodiments, the branched-chain esters are a base oil, such as an API Group V base oil, that can be blended with a diluent. Any suitable diluent can be used. In some embodiments, the diluent is an API Group I base oil, an API Group II base oil, an API Group III base oil, an API Group IV base oil, another API Group V base oil, or any combination thereof. In some embodiments, the diluent comprises a poly-alpha-olefin (PAO), such as hydrogenated polydecene. In some embodiments, the diluent is an API Group II base oil, an API Group III base oil, an API Group IV base oil, or a mixture thereof. In some such embodiments, the diluent is an API Group II base oil. In some other such embodiments, the diluent is an API Group III base oil. In some further such embodiments, the diluent is an API Group IV base oil.

The branched-chain ester can be included in the lubricant composition in any suitable amount. For example, in some embodiments, the weight-to-weight ratio of the branched-chain esters to the diluent is from 1:50 to 5:1, or from 1:20 to 2:1, or from 1:10 to 1:1. In some other embodiments, the branched chain ester is present in the lubricant composition in an amount no more than 60 percent by weight, or 50 percent by weight, or 40 percent by weight, or 30 percent by weight, or 20 percent by weight, or 10 percent by weight, based on the total weight of the lubricant composition. In some such embodiments, the branched chain ester is present in the lubricant composition in an amount of at least 1 percent by weight, or at least 5 percent by weight, or at least 10 percent by weight, or at least 15 percent by weight, based on the total weight of the lubricant composition.

Such lubricant compositions can be formed in any suitable manner. For example, in some embodiments, a method of making the lubricant composition includes: providing the branched-chain ester(s); and optionally combining the branched-chain ester(s) with at least one other material, such as a diluent.

Such method may produce a lubricant composition from renewable feedstocks, and may advantageously provide simpler and/or more cost-effective production, reduced variability, improved sourcing, and increased biorenewability than conventional methods for producing a lubricant composition from petrochemical feedstocks. In addition, lubricant compositions formed by such methods may have useful combinations of properties, including but not limited to, high viscosity index, oxidative stability, thermal stability, and hydrolytic stability.

In some embodiments, the one or more additional ingredients can include one or more additives, such as those typically used in lubricant compositions. Such additives include, but are not limited to, dispersants, detergents, antiwear agents, antioxidants, metal deactivators, extreme pressure (EP) additives, viscosity modifiers such as viscosity index improvers, pour point depressants, corrosion inhibitors, friction coefficient modifiers, colorants, antifoam agents, antimisting agents, demulsifiers, organomolybdenum compounds, and zinc dialkyl dithiophosphates. In some embodiments, for example, where the lubricant composition is blended to be suitable for use as a gear oil, the lubricant composition comprises a standard additive package, such as an additive package for a GL-4 or GL-5 gear oil.

The one or more additives can be used in any suitable amount in the lubricant composition. The quantity and combination of additives used can depend on a variety of factors, including, but not limited to, the properties of the base oil, the properties of the selected additives, and the desired properties of the resulting composition. In some embodiments, the one or more additives make up from 0.1 to 50 weight percent, or from 0.1 to 40 weight percent, or from 0.1 to 30 weight percent, or from 0.1 to 20 weight percent, or from 0.1 to 15 weight percent.

The lubricant compositions disclosed herein can be employed in a variety of contexts. Non-limiting examples include, but are not limited to, motor oils, transmission fluids, gear oils, industrial lubricating oils, metalworking oils, hydraulic fluids, drilling fluids, greases, compressor oils, cutting fluids and milling fluids. In some embodiments, the lubricant compositions can be used for lubricating an internal combustion engine, a diesel engine, a two-cycle engine, a crankcase, a gearbox, one or more bearings, or a transmission.

Thus, in certain aspects, the disclosure provides methods of lubricating various mechanical systems, the methods comprising supplying to the mechanical system a lubricant composition comprising an alpha-olefin copolymer compositions according to any of the above embodiments.

In some embodiments, the disclosure provides methods of lubricating a transmission, a differential, or a transfer case, the method comprising supplying to a transmission, a differential, or a transfer case, a lubricant composition comprising branched-chain esters, as disclosed herein.

In some embodiments, the disclosure provides methods of lubricating an internal combustion engine, the method comprising supplying to an internal combustion engine a lubricant composition comprising branched-chain esters, as disclosed herein.

In some embodiments, the disclosure provides methods of lubricating a diesel engine, the method comprising supplying to a diesel engine a lubricant composition comprising branched-chain esters, as disclosed herein.

### Personal Care Compositions, and Methods of Making and Using the Same

In certain aspects, the disclosure provides personal care compositions that include branched-chain esters according to any of the above embodiments. In some embodiments, the personal care compositions contain one or more additional ingredients.

The branched-chain esters can be incorporated into any suitable personal care composition, including, but not limited to, compositions suitable for application to human skin and/or hair. Non-limiting examples of such compositions are shampoos, soaps, conditioners, moisturizers, creams, lotions, emollients, hair products, cosmetics, and sunscreens.

In some embodiments, the personal care composition further includes a diluent. In some embodiments, the diluent includes a hydrophilic material, such as water or glycerin. In some such embodiments, the personal care composition is an emulsion. In some other embodiments, the diluent is a hydrophobic or oleaginous material. Non-limiting examples of hydrophobic or oleaginous diluents are poly(alpha-olefin), mineral oil, petrolatum, ester lipids, silicone lipids, or any mixtures thereof.

The branched-chain ester can be included in the personal care composition in any suitable amount. For example, in some embodiments, the weight-to-weight ratio of the branched-chain esters to the diluent is from 1:50 to 5:1, or from 1:20 to 2:1, or from 1:10 to 1:1. In some other embodiments, the branched chain ester is present in the personal care composition in an amount no more than 60 percent by weight, or 50 percent by weight, or 40 percent by weight, or 30 percent by weight, or 20 percent by weight, or 10 percent by weight, based on the total weight of the personal care composition. In some such embodiments, the branched chain ester is present in the personal care composition in an amount of at least 1 percent by weight, or at least 5 percent by weight, or at least 10 percent by weight, or at least 15 percent by weight, based on the total weight of the personal care composition.

In some embodiments, the personal care compositions include one or more additional ingredients. Such additional ingredients include, but are not limited to, emollients, moisturizers, conditioners, oils, sunscreens, surfactants, emulsifiers, preservatives, rheology modifiers, colorants, preservatives, pH adjustors, propellants, reducing agents, fragrances, foaming or de-foaming agents, tanning agents, depilatory agents, astringents, antiseptics, deodorants, antiperspirants, insect repellants, bleaches, tighteners, anti-dandruff agents, adhesives, polishes, strengtheners, fillers, barrier materials, and biocides.

### EXAMPLES

The following Examples illustrate certain aspects and embodiments of the compounds, compositions, and methods disclosed herein. The Examples merely illustrate particular embodiments and aspects of the disclosed subject matter, and are not intended to provide substantive limits on the scope of the claimed subject matter.

### Example 1 - Monoester of Unsaturated Polvdecene

An unsaturated polydecene **1A** is reacted with decanoic acid **1B** in the presence of trifluoromethanesulfonic acid (TfOH) to yield a branched ester **1C**.

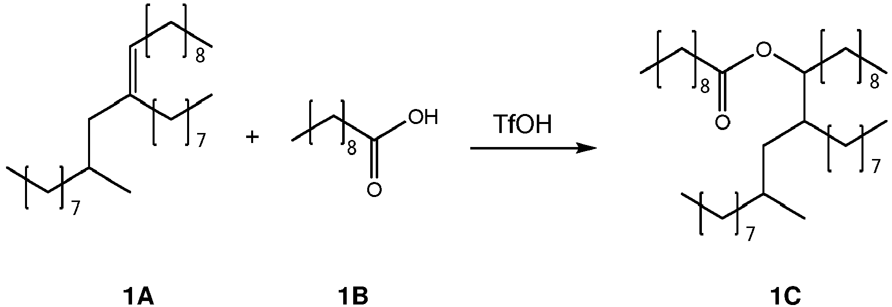

Into a two-necked round-bottom flask was added 75.0 g of the unsaturated polydecene **1A** and 106.0 g of decanoic acid **1B**. The flask was flushed with nitrogen gas and heated to 50 °C. Then, 5 g of TfOH was added via pipet, which caused the solution to turn to an amber color. The flask was heated to 100 °C under nitrogen gas for 20 hours. The temperature was reduced to 60 °C for 4 hours, and then reduced to room temperature. The reaction product was diluted with ethyl acetate (200 mL) and quenched with potassium hydroxide (1 M, 100 mL). The quenched product was then transferred to a separatory funnel and washed with potassium hydroxide (1 M, 4 x 100 mL) and brine (2 x 100 mL). The washed product was dried over magnesium sulfate, filtered, and concentrated in vacuo to give a golden yellow oil (72 g).

The product was analyzed by infrared spectroscopy, which showed a carbonyl stretch at 1736 cm⁻¹, which is consistent with the formation of an estolide bond. The kinematic viscosity (ASTM D445) of the oil was 21.3 cSt at 40°C and 4.6 cSt at 100 °C, and the viscosity index (ASTM D2270) was 135. The product showed an aniline point (ASTM D611) of 109 °C and an iodine value (ASTM D5554) of 90.

### Example 2 - Monoester of 3-Dodecene

Palmitic acid (85%) was reacted with 3-dodecene in the presence of sulfuric acid. The acid mixture and 3-dodecene were added to a 500-mL 4-neck round-bottom flask. Sulfuric acid (5% by vol., 5 mL) was added to the flask, and the reaction mixture was heated to 55 °C under nitrogen gas for 18 hours. The reaction mixture was cooled to room temperature, and 2-ethylhexanol (64 mL) was added. The mixture was then heated to 55 °C at 10 torr-g for 2 hours in a flask equipped with a short distillation path for the collection of generated water. The reaction mixture was allowed to cool to room temperature. A solution of potassium hydroxide (2.1 equiv. with respect to H₂SO₄) was added to the cooled solution, and the organic layer was separated and washed with brine, dried over magnesium sulfate, and filtered into a pre-weighed 500-mL two-neck round-bottom flask. The reaction mixture was heated gradually (over 2 hours) to 170 °C to distill any unreacted 3-dodecene and 2-ethylhexanol away from the reaction product. The reaction product was filtered and analyzed by gas chromatography and infrared spectrophotometry. The analyses confirmed the formation of major product of 3(4)-dodecyl palmitate and a minor amount of 2-ethylhexyl palmitate. The kinematic viscosity (ASTM D445) of the resultant oil was 3.4 cSt at 100 °C and 12.4 cSt at 40 °C, and the viscosity index (ASTM D2270) was 151.

### Example 3 - Monoester of 9-Octadecene

Decanoic acid (1:1) is reacted with 9-octadecene in a manner analogous to that shown in Example 2 to yield 1-octyldecyl decanoate.
The present application is a divisional application based on an earlier European Patent Application No 15751927.3, which was derived from PCT Application No PCT/US2015/014384. The following numbered clauses, which correspond to the claims of that earlier PCT Application as filed, form part of the present disclosure and in particular form further aspects of the invention, whether or not they appear in the presently appended claims.
1. A compound of formula (I): 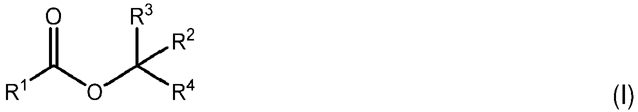 wherein:
   R¹ is C₃₋₂₄ alkyl or C₃₋₂₄ alkenyl, each of which can be optionally substituted one or more times by substituents selected independently from R⁵;
   R² is a hydrogen atom or C₁₋₆ alkyl, which can be optionally substituted one or more times by substituents selected independently from R⁵;
   R³ and R⁴ are independently C₃₋₂₄ alkyl or C₃₋₂₄ alkenyl, each of which can be optionally substituted one or more times by substituents selected independently from R⁵; and
   R⁵ is a halogen atom, -OH, -NH₂, C₁₋₆ alkyl, C₁₋₆ heteroalkyl, C₂₋₆ alkenyl, C2-6 heteroalkenyl, C₃₋₁₀ cyclokalkyl, or C₂₋₁₀ heterocycloalkyl.
2. The compound of clause 1, wherein R¹ is C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.
3. The compound of clause 1, wherein R¹ is C₄₋₂₄ alkyl or C₃₋₂₄ alkenyl, each of which is optionally substituted one or more times with -OH.
4. The compound of clause 1, wherein R¹ is hexyl, heptyl, octyl, nonyl, decyl, undecyl, dodecyl, tridecyl, tetradecyl, pentadecyl, hexadecyl, or heptadecyl.
5. The compound of clause 1, wherein R¹ is nonyl or undecyl.
6. The compound of any one of clauses 1 to 5, wherein R² is hydrogen.
7. The compound of any one of clause 1 to 6, wherein R³ and R⁴ are independently C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.
8. The compound of clause 7, wherein R³ and R⁴ are independently C₃₋₂₄ alkyl or C₃₋₂₄ alkenyl, each of which is optionally substituted one or more times with -OH.
9. The compound of clause 7, wherein R³ and R⁴ are independently propyl, butyl, pentyl, hexyl, heptyl, octyl, nonyl, decyl, undecyl, dodecyl, tridecyl, tetradecyl, pentadecyl, hexadecyl, or heptadecyl.
10. The compound of clause 7, wherein R³ and R⁴ are independently propyl, butyl, pentyl, hexyl, heptyl, octyl, nonyl, or decyl.
11. The compound of clause 7, wherein one of R³ and R⁴ is octyl or nonyl.
12. The compound of clause 7, wherein one of R³ and R⁴ is octyl and the other is nonyl.
13. A lubricant composition, comprising a composition of any one of clauses 1 to 12.
14. The lubricant composition of clause 13, further comprising a diluent.
15. The lubricant composition of clause 14, wherein the diluent comprises a Group I base oil, a Group II base oil, a Group III base oil, a Group IV base oil, a Group V base oil, or a mixture thereof.
16. The lubricant composition of clause 14, wherein the diluent comprises a poly(alpha-olefin).
17. The lubricant composition of any one of clauses 14 to 16, wherein the weight-to-weight ratio of the compound to the diluent is from 1:50 to 5:1.
18. The lubricant composition of any one of clauses 14 to 17, further comprising one or more additives.
19. The lubricant composition of clause 18, where the one or more additives comprise one or more dispersants, one or more antiwear agents, one or more antioxidants, one or more metal deactivators, one or more extreme pressure additives, one or more dispersants, one or more viscosity modifiers, one or more pour point depressants, one or more corrosion inhibitors, one or more friction coefficient modifiers, one or more colorants, one or more antifoam agents, one or more antimisting agents, one or more demulsifiers, or any combinations thereof.
20. The lubricant composition of any one of clauses 13 to 19, which is adapted for lubricating an internal combustion engine, a diesel engine, a two-cycle engine, a crankcase, a gearbox, one or more bearings, or a transmission.
21. A method for making a lubricant composition, the method comprising:
   providing a compound of any one of clauses 1 to 12 and a diluent, where the diluent is miscible with the compound; and
   mixing the compound and the diluent to form a diluted composition.
22. The method of clauses 21, wherein the diluent comprises a Group I base oil, a Group II base oil, a Group III base oil, a Group IV base oil, a Group V base oil, or a mixture thereof.
23. The method of clauses 22, where the diluent comprises a poly(alpha-olefin).
24. The method of any one of clauses 21 to 23, further comprising: providing one or more additives; and mixing the one or more additives and the diluted composition.
25. The method of clauses 24, where the one or more additives comprise one or more detergents, one or more antiwear agents, one or more antioxidants, one or more metal deactivators, one or more extreme pressure additives, one or more dispersants, one or more viscosity modifiers, one or more pour point depressants, one or more corrosion inhibitors, one or more friction coefficient modifiers, one or more colorants, one or more antifoam agents, one or more antimisting agents, one or more demulsifiers, or any combinations thereof.
26. A method of lubricating an apparatus, comprising:
   introducing to an apparatus a lubricant composition of any one of clauses 13 to 20.
27. The method of clause 26, wherein the apparatus is an internal combustion engine, a diesel engine, a two-cycle engine, a crankcase, a gearbox, one or more bearings, or a transmission.
28. A personal care composition, comprising a compound of any one of clause 1 to 12.
29. The personal care composition of clause 28, further comprising a diluent.
30. The personal care composition of clause 29, wherein the diluent is water.
31. The personal care composition of clause 30, wherein the composition is an emulsion.
32. The personal care composition of clause 29, wherein the diluent is a hydrophilic material.
33. The personal care composition of clause 32, wherein the hydrophilic material is glycerin.
34. The personal care composition of clause 29, wherein the diluent is an oleaginous material.
35. The personal care composition of clause 34, wherein the diluent a poly(alpha-olefin), mineral oil, petrolatum, ester lipids, silicone lipids, or any mixtures thereof.
36. The personal care composition of any one of clauses 29 to 35, wherein the weight-to-weight ratio of the compound to the diluent is from 1:50 to 5:1.
37. The personal care composition of any one of clauses 28 to 36, further comprising one or more additives.
38. The personal care composition of clauses 37, where the one or more additives comprise emollients, one or more moisturizers, one or more conditioners, one or more oils, one or more sunscreens, one or more surfactants, one or more emulsifiers, one or more preservatives, one or more rheology modifiers, one or more colorants, one or more preservatives, one or more pH adjustors, one or more propellants, one or more reducing agents, one or more fragrances, one or more foaming or one or more de-foaming agents, one or more tanning agents, one or more depilatory agents, one or more astringents, one or more antiseptics, one or more deodorants, one or more antiperspirants, one or more insect repellants, one or more bleaches, one or more tighteners, one or more anti-dandruff agents, one or more adhesives, one or more polishes, one or more strengtheners, one or more fillers, one or more barrier materials, one or more biocides, or any combinations thereof.
39. The personal care composition of any one of clauses 28 to 38, which is adapted for application to human skin or hair.

Branched-chain esters and methods of making branched-chain esters are generally disclosed. Various uses of such compounds are also disclosed, including uses in personal care compositions and lubricant compositions. In some embodiments, the branched-chain esters are at least partially derived from a renewable source, such as a natural oil.

## Claims

1. A compound of formula (I): 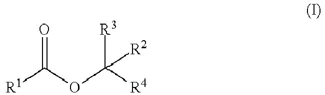 wherein:
R¹ is C₃₋₂₄ alkyl or C₃₋₂₄ alkenyl, each of which can be optionally substituted one or more times by substituents selected independently from R⁵;
R² is a hydrogen atom or C₁₋₆ alkyl, which can be optionally substituted one or more times by substituents selected independently from R⁵;
R³ and R⁴ are independently C₃₋₂₄ alkyl or C₃₋₂₄ alkenyl, each of which can be optionally substituted one or more times by substituents selected independently from R⁵; and
R⁵ is a halogen atom, -OH, -NH₂, C₁₋₆ alkyl, C₁₋₆ heteroalkyl, C₂₋₆ alkenyl, C₂₋₆ heteroalkenyl, C₃₋₁₀ cyclokalkyl, or C₂₋₁₀ heterocycloalkyl.

2. The compound of claim 1, wherein R¹ is C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, -NH(C₁₋₆ alkyl), and -N(C₁₋₆ alkyl)₂.

3. The compound of claim 1, wherein R¹ is C₄₋₂₄ alkyl or C₃₋₂₄ alkenyl, each of which is optionally substituted one or more times with -OH.

4. The compound of claim 1, wherein R¹ is hexyl, heptyl, octyl, nonyl, decyl, undecyl, dodecyl, tridecyl, tetradecyl, pentadecyl, hexadecyl, or heptadecyl.

5. The compound of claim 1, wherein R¹ is nonyl or undecyl.

6. The compound of claim 1, wherein R² is hydrogen.

7. The compound of claim 1, wherein R³ and R⁴ are independently C₃₋₂₄ alkyl or C₃₋₂₄ alkenyl, each of which can be optionally substituted one or more times by substituents selected independently from the group consisting of: a halogen atom, -OH, -O(C₁₋₆ alkyl), -NH₂, - NH(C₁₋₆ alkyl), and -N(C₁₋₆ alkyl)₂.

8. The compound of claim 7, wherein R³ and R⁴ are independently C₃₋₂₄ alkyl or C₃₋₂₄ alkenyl, each of which is optionally substituted one or more times with -OH.

9. The compound of claim 7, wherein R³ and R⁴ are independently propyl, butyl, pentyl, hexyl, heptyl, octyl, nonyl, decyl, undecyl, dodecyl, tridecyl, tetradecyl, pentadecyl, hexadecyl, or heptadecyl.

10. The compound of claim 7, wherein R³ and R⁴ are independently propyl, butyl, pentyl, hexyl, heptyl, octyl, nonyl, or decyl.

11. The compound of claim 7, wherein one of R³ and R⁴ is octyl and the other is nonyl.

12. A lubricant composition, comprising a compound of claim 1, further comprising a diluent.

13. The lubricant composition of claim 12, wherein the diluent comprises a Group I base oil, a Group II base oil, a Group III base oil, a Group IV base oil, a Group V base oil, or a mixture thereof, wherein the diluent comprises a poly(alpha-olefin).

14. The lubricant composition of claim 12, wherein the weight-to-weight ratio of the compound to the diluent is from 1:50 to 5:1.

15. The lubricant composition of claim 12, further comprising one or more additives, wherein the one or more additives comprise one or more dispersants, one or more antiwear agents, one or more antioxidants, one or more metal deactivators, one or more extreme pressure additives, one or more dispersants, one or more viscosity modifiers, one or more pour point depressants, one or more corrosion inhibitors, one or more friction coefficient modifiers, one or more colorants, one or more antifoam agents, one or more antimisting agents, one or more demulsifiers, or any combinations thereof.
